# EUROPÄISCHE PATENTANMELDUNG

(11) **EP 1 818 314 A2**
(43) Veröffentlichungstag der Anmeldung: **15.08.2007**
(21) Anmeldenummer: 07075030.2
(22) Anmeldetag: 12.01.2007
(51) Int. Cl.: C02F 3/28

(54) **Verfahren und Biogasanlage zur Herstellung von Biogas aus organisch beladenen Flüssigkeiten**

(30) Priorität: 13.02.2006 DE 102006006743
(71) Anmelder: Brandenburgische Technische Universität Cottbus, 03046 Cottbus (DE)
(72) Erfinder: Busch, Günter, Prof.,Dr.-Ing., 01462 Cossebaude (DE); Grossmann, Jochen, Dr.-Ing., 01187 Dresden (DE); Yinnavong, Ninaphone, Dipl.-Ing., 03044 Cottbus (DE)
(74) Vertreter: Schubert, Klemens

(57) **Zusammenfassung**

Verfahren und Biogasanlage zur Herstellung von Biogas aus organisch beladenen Flüssigkeiten, wobei
a) man die organisch beladenen Flüssigkeiten in einem Mischbehälter belüftet und anschließend in einen Hydrolysefestbettreaktor überführt,
b) man eine Hydrolyse in mindestens einem Hydrolysefestbettreaktor durchführt, der Füllkörper enthält, wobei man die Flüssigkeit mehrfach über die Füllkörper führt indem man die Flüssigkeit abpumpt und über einen Rückführungskreislauf wieder dem Hydrolysefestbettreaktor zuführt oder die Flüssigkeit in weitere Hydrolysefestbettreaktoren pumpt,
c) man einen Teilstrom der organisch beladenen Flüssigkeit aus dem Rückführungskreislauf in einen Methanfestbettreaktor pumpt, der Füllkörper enthält und zu Biogas vergärt.

## Beschreibung

Die Erfindung betrifft ein Verfahren zur Herstellung von Biogas aus organisch beladenen Flüssigkeiten und eine dazugehörige Biogasanlage sowie deren Verwendung.

Abwässer mit organischen, bioverfügbaren Inhaltstoffen, deren Konzentration üblicherweise durch den chemischen Sauerstoffbedarf (CSB) für chemisch oxidierbare Stoffe oder durch den biologischen Sauerstoffbedarf (BSB) für biologisch oxidierbare Stoffe angegeben wird, werden in der Regel aerob behandelt, d.h. mit Hilfe von Mikroorganismen unter der Zufuhr von Luftsauerstoff biologisch in die Hauptendprodukte, Kohlendioxid und Wasser umgewandelt. Bioverfügbare Stoffe sind solche, die durch mikrobielle, extra- oder intrazelluläre Prozesse um- oder abgebaut werden können.

Aerobe Verfahren, d.h. biologische Oxidation organischer Abwasserinhaltsstoffe unter Zufuhr von Luftsauerstoff, bedeuten aus energetischer Sicht einen Energieverlust, da die freiwerdende Wärme der biochemischen Prozesse aufgrund des niedrigen Temperaturniveaus nicht technisch genutzt werden kann.

Grundsätzlich kann das Abwasser auch anaerob, d. h. unter Ausschluss von Luftsauerstoff, unter der Wirkung von Mikroorganismen in Biogas (Kohlendioxid und Methan) umgewandelt werden. Da übliche Biogasverfahren sehr empfindlich auf Änderungen der Zusammensetzung des Inputmaterials oder der Prozessbedingungen reagieren, können organisch hoch belastete Abwässer zur Gewährleistung der Prozessstabilität nur in geringen Mengen den Hauptsubstraten, üblicherweise tierische Exkremente, als so genannte Co-Substrate zugesetzt werden.

Es ist bekannt, dass die Umwandlung von organischen Stoffen in Biogas über mehrere biochemische Schritte erfolgt, die in Hydrolyse und Methanisierung eingeteilt werden können. In der Hydrolyse werden aus den organischen Stoffen wasserlösliche Monomere und niedere organische Säuren gebildet, die in der Methanisierung dann in Methan umgewandelt werden. Beide Prozesse finden in den einstufigen Verfahren zeitlich und räumlich nebeneinander statt, in zweistufigen Verfahren finden Hydrolyse und Methanisierung in apparate- und prozesstechnisch getrennten Stufen statt.

Bisher wurde die Hydrolyse als getrennte Prozessstufe nur für organische feste und flüssige Substrate, wie zum Beispiel nachwachsende Rohstoffe und Gülle, beschrieben. Eine hydrolytische Spaltung von Abwasser zum Zweck der Vergärung wurde bisher nicht praktiziert. Des Weiteren wurde bisher die Hydrolyse von flüssigen Substraten ausschließlich in der flüssigen Phase durchgeführt.

Der Erfindung liegt die Aufgabe zugrunde Biogas aus mit organisch beladenen Flüssigkeiten herzustellen.

Die Aufgabe wird durch ein Verfahren gelöst, bei dem
a) man die organisch beladenen Flüssigkeiten in einem Mischbehälter belüftet und anschließend in einen Hydrolysefestbettreaktor überführt,
b) man eine Hydrolyse in mindestens einem Hydrolysefestbettreaktor durchführt, der Füllkörper enthält, wobei man die Flüssigkeit mehrfach über die Füllkörper führt indem man die Flüssigkeit abpumpt und über einen Rückführungskreislauf wieder dem Hydrolysefestbettreaktor zuführt oder die Flüssigkeit in weitere Hydrolysefestbettreaktoren pumpt,
c) man einen Teilstrom der organisch beladenen Flüssigkeit aus dem Rückführungskreislauf in einen Methanfestbettreaktor pumpt, der Füllkörper enthält und zu Biogas vergärt.

Das Verfahren dient der energetischen Nutzung von organischen beladenen Flüssigkeiten, wie zum Beispiel Abwässern, durch Erzeugung von Biogas. Dabei wird die chemisch gebundene Energie der organischen, bioverfügbaren Inhaltsstoffe von Abwässern für die Herstellung von Biogas genutzt.

Hierzu wird ein zweistufiges, aus einer Hydrolysestufe und einer Methanstufe bestehendes Biogasverfahren genutzt.

Somit stellt das erfindungsgemäße Verfahren ein prozessstabiles Verfahren für die anaerobe mikrobielle Umwandlung der bioverfügbaren organischen Inhaltsstoffe von zum Beispiel Abwässern dar.

Sind die organischen Inhaltsstoffe der Abwässer sehr leicht biologisch abbaubar, so kann es zur raschen Bildung großer Mengen an organischen Säuren kommen. Als Ergebnis dessen "kippen" gebräuchliche Biogasverfahren um, d.h. es kommt zur Versäuerung und somit zur Abtötung bzw. Inhibierung der Methanbakterien, wodurch die Biogasproduktion zusammenbricht. Daher konnten bisher solche Stoffe nur in sehr geringen Anteilen dem Hauptsubstrat der Biogasanlage zugemischt werden.

Der erreichte Vorteil dieses Verfahrens besteht darin, dass auch solche organisch belasteten Abwässer, die wegen der vorstehend genannten Gründe in Biogasanlagen herkömmlicher Verfahrensweise gar nicht oder nur in geringen Anteilen zur Biogaserzeugung eingesetzt werden konnten, nunmehr in sehr hohen Anteilen oder als alleiniges Substrat verwendet werden können. Dadurch wird es möglich, grundsätzlich den Gehalt an (bio-)chemischer Energie jedes Abwassers für die Biogasgewinnung zu nutzen. Enthält das Abwasser außerdem noch feste Bestandteile, so können diese vorher abgetrennt werden.

Die organisch beladene Flüssigkeit versetzt man bevorzugt im Mischbehälter mit Kreislaufwasser.

Hierzu pumpt man vorzugsweise das Kreislaufwasser aus dem Ablauf des Methanfestbettreaktors in den Mischbehälter.

Die Mikroorganismenpopulation in der Hydrolyse- und in der Methanstufe benötigen neben den abzubauenden organischen Stoffen auch mineralische Nährstoffe und Spurenelemente. Deren Konzentration können in der erforderlichen Größe aufrechterhalten werden, wenn zumindest ein Teil des den Methanreaktor verlassenden Abwassers wieder in die Hydrolyse als Kreislaufwasser zurückgeführt wird. Mit dieser Rückführung werden auch Mikroorganismen rückgeführt, die für die ständige Wiederbeimpfung der Hydrolyse sorgen. Das Kreislaufwasser kann auch zur Verdünnung des organisch belasteten Abwassers genutzt werden, falls dieses eine zu hohe Eingangskonzentration aufweisen sollte.

Als Füllkörper verwendet man vorzugsweise Holzhackschnitzel und/oder Kunststofffüllkörper und/oder Bambusringe. Es können aber andere Arten von Füllkörper verwendet werden.

Die Füllkörper in den Hydrolysefestbettreaktoren beimpft man bevorzugt mit Hydrolysebakterien.

Die Füllkörper in den Methanfestbettreaktoren beimpft man bevorzugt mit Methanbakterien. Das in der Hydrolyse gebildete Hydrolysat wird in eine Methanstufe geführt, die als Festbettreaktor gestaltet und mit Füllkörpern, auf denen die Methanbakterien immobilisiert sind, ausgestattet ist. Es wurde herausgefunden, dass diese Ausführungsform in besonderem Maße für die Methanisierung des in der Hydrolyse gebildeten Hydrolysats geeignet ist, insbesondere dann, wenn die Konzentration der organischen Säuren sehr hoch ist. Methanstufen in anderen Ausführungsformen versagen regelmäßig bei hohen Säurekonzentrationen des Hydrolysats. Des Weiteren können hier sehr kurze hydraulische Verweilzeiten im Bereich von wenigen Stunden realisiert werden, da die methanbildenden Mikroorganismen immobilisiert sind und nicht mehr ausgespült werden können. Durch die Steuerung der Zufuhr des Hydroysats in diese Art des Methanreaktors ist auch die Biogasproduktion steuerbar.

Die Hydrolysefestbettreaktoren betreibt man vorzugsweise als offenes System, so dass das bei der Hydrolyse gebildete Kohlendioxid (CO₂) in die Atmosphäre entweichen kann. In der Hydrolyse werden grundsätzlich größere Mengen an Kohlendioxid gebildet. Wenn dieses Gas mit dem in der Methanstufe gebildeten Methan zusammengeführt wird, so wirkt es verdünnend, d.h. die Methankonzentration und somit der Brennwert des Biogases vermindern sich. Gestaltet man die Hydrolyse verfahrenstechnisch und apparatetechnisch so, dass das Kohlendioxid bereits hier entweichen kann, so erhält man eine höhere Konzentration an Methan im Biogas. Das Biogas hat dann einen höheren Brennwert und lässt sich mit höherem Wirkungsgrad verwerten.

Das die Hydrolysestufe im Hydrolysefestbettreaktor verlassende Hydrolysat besteht aus dem Hauptbestandteil Wasser und den biochemisch in organische Säuren und andere niedere, wasserlösliche organische Verbindungen gespaltenen organischen Bestandteilen, die dem Methanfestbettreaktor zugeführt werden.

Die Hydrolyse im Hydrolysefestbettreaktor betreibt man bevorzugt unter schwach aeroben Bedingungen. In der Praxis lässt sich der Eintrag von methanbildenden Mikroorganismen in der Hydrolyse nicht vollständig vermeiden, so dass es auch in der Hydrolyse zur Bildung von Methan kommen kann. Wenn die Hydrolyse offen betrieben wird, so könnte das einen Verlust an Methan bedeuten und gleichzeitig erhöhte Anforderungen an den Explosionsschutz stellen. Die Methanbildung in der Hydrolyse kann jedoch vermieden werden, wenn man den Zutritt von geringen Mengen Luftsauerstoff gestattet. Die Methanbakterien werden durch den Sauerstoff abgetötet oder inhibiert, so dass keine oder nur eine vernachlässigbar geringe Methanbildung stattfindet. Außerdem kann die Überlagerung mit aeroben Abbauprozessen zu einer höheren Ausbeute führen.

Weiterhin wird die Aufgabe der vorliegenden Erfindung durch eine Biogasanlage, bestehend aus einem Mischbehälter mit Belüftung, mindestens einem Hydrolysefestbettreaktor, Pumpen und einem Methanfestbettreaktor gelöst. Mit dieser Biogasanlage wird die energetische Nutzung von organische beladenen Flüssigkeiten wie zum Beispiel organisch belastete Abwässer, durch Erzeugung von Biogas ermöglicht. Die organischen Bestandteile der Abwässer werden dabei in einem zweistufigen, aus einer Hydrolysestufe und einer Methanstufe bestehenden Prozess weitestgehend in Biogas umgewandelt.

Die Hydrolysestufe wird in einem offenen, ein- oder mehrstufigen Hydrolysefestbettreaktor durchgeführt, auf dessen Füllkörpern Hydrolysebakterien immoblisiert sind und der ein- oder mehrfach von dem feststofffreien und zu verwertendem Abwasser durchströmt wird. Das Hydrolysat wird der Methanstufe zugeführt, in der sich auf Füllkörpern immobilisierte Methanbakterien befinden und in der verwertbares Biogas produziert wird. Der Ablauf aus diesem Methanreaktor kann teilweise in die Hydrolysestufe nach Belüftung zurückgeführt werden, der Rest wird entsorgt.

Bevorzugt sind mehrere Hydrolysefestbettreaktoren in Reihe hintereinander betreibar. Üblicherweise wird die Hydrolyse als einstufiger Festbettreaktor mit Füllkörpern oder anderen festen Einbauten ausgeführt, über die das zu hydrolysierende Abwasser ein- oder mehrfach mit Hilfe einer Umwälzeinrichtung geführt wird. Es kann auch zur Erreichung einer höheren Ausbeute bzw. eines höheren biochemischen Umsatzes sinnvoll sei, mehrere Hydrolysereaktoren in strommäßiger Reihenschaltung oder als so genanntes Reaktionsrohr zu betreiben.

Die Hydrolyse kann daher auch mehrstufig als Reihenschaltung mehrer Hydrolysefestbettreaktoren oder als so genannter Rohrreaktor betrieben werden.

Die Hydrolysefestbettreaktoren enthalten vorzugsweise Füllkörper, auf denen Hydrolysebakterien immobilisiert sind.

Der Methanfestbettreaktor enthält vorzugsweise Füllkörper, auf denen Methanbakterien immobilisiert sind.

Die Füllkörper sind bevorzugt Holzhackschnitzel und/oder Kunststofffüllkörper und/oder Bambusringe.

Bevorzugt verbindet ein Leitungssystem mit Pumpen (Förderpumpen) den Mischbehälter, die Hydrolysereaktoren und den Methanreaktor miteinander. Durch ein solches Leitungssystem wird die Möglichkeit geschaffen, das aus dem ersten Hydrolysefestbettreaktor erhaltene Hydrolysat in einen weiteren Hydrolysefestbettreaktor oder nach mehreren Hydrolysestufen in den Methanfestbettreaktor zu pumpen.

Die Hydrolysefestbettreaktoren enthalten vorzugsweise einen Rückführungskreislauf mit einer Pumpe (Rückführungspumpe). Durch diesen Rückführungskreislauf kann die dem Hydrolysefestbettreaktor entzogene organisch beladene Flüssigkeit wieder in den Hydrolysefestbettreaktor zurückgepumpt werden, so dass die organisch beladenen Flüssigkeit durch einen Hydrolysefestbettreaktor zyklisiert werden kann.

Der Methanfestbettreaktor besitzt vorzugsweise einen Ablauf. Aus diesem Ablauf kann das nach der Biogasreaktion übrig bleibende entfrachtetes Abwasser dem System entzogen werden oder als Kreislaufwasser wieder in das System zurückgepumpt werden.

Der Methanfestbettreaktor besitzt vorzugsweise einen Kreislauf für Kreislaufwasser von dem Methanfestbettreaktor zum Mischbehälter mit einer Pumpe (Kreislaufpumpe). Durch diesen Kreislauf kann das aus dem Ablauf des Methanfestbettreaktors entzogene Kreislaufwasser wieder in den Mischbehälter gepumpt und belüftet werden. Es hat sich als vorteilhaft erwiesen, wenn nicht nur die Hydrolyse schwach aerob betrieben wird, sondern auch das rück geführte Kreislaufwasser vor seinem Eintritt in die erste Hydrolysestufe belüftet wird, um die mitgeschleppten methanbildenden Mikroorganismen abzutöten oder zu inhibieren.

Des Weiteren wird die Aufgabe der vorliegenden Erfindung durch eine Verwendung der Biogasanlage zur Herstellung von Biogas gelöst.

Bei der Herstellung von Biodiesel, z.B. des Rapsöl-Methyl-Ester (RME), fällt Glyzerin in einer Menge von ca. 10 % des eingesetzten Rapsöls an. Dieses Glyzerin findet sich in hoher Konzentration im Abwasser des Herstellungsprozesses oder bildet sein Nebenprodukt. Es ist bekannt, dass diese Glyzerin beladenen Abwasser in Biogasanlagen einen hohen Biogasertrag hervorbringen, allerdings kann es nur bis zu einem Anteil von ca. 3 % dem Regelsubstrat der Vergärungsanlage zugesetzt werden, da es sonst zum Prozessversagen kommen kann. Es ist nun möglich, dieses Glyzerin beladene Abwasser einer erfindungsgemäßen Biogasanlage ohne jegliche Zumischung im Sinne einer MonoVergärung zuzuführen und daraus sehr große Mengen Biogas mit einem Methangehalt von mehr als 60 % herzustellen. Die Umwandelung des Glyzerin beladenen Abwassers in Biogas ist insofern wirtschaftlich bedeutsam, da mit steigender Produktion an Biodiesel andere Verwertungswege zunehmend gesättigt sind.

Ferner wird die Aufgabe der vorliegenden Erfindung durch die Verwendung des Verfahrens zur Monovergärung von glyzerinhaltigem Abwasser gelöst. Das Verfahren ist insbesondere für die Monovergärung von glyzerinhaltigem Abwasser aus der Biodiesel-Produktion geeignet.

Das Verfahren der Biogasgewinnung aus Abwässern, die organische, bioverfügbare Stoffe enthalten, erschließt eine weitere, bisher wenig genutzte Ressource zur Gewinnung eines hochwertigen Energieträgers. Es erlaubt die Verwendung geeigneter industrieller oder kommunaler Abwässert ohne deren Mischung mit anderen Stoffen, wenn die Nährstoffanforderungen der beteiligten Mikroorganismen erfüllt werden.

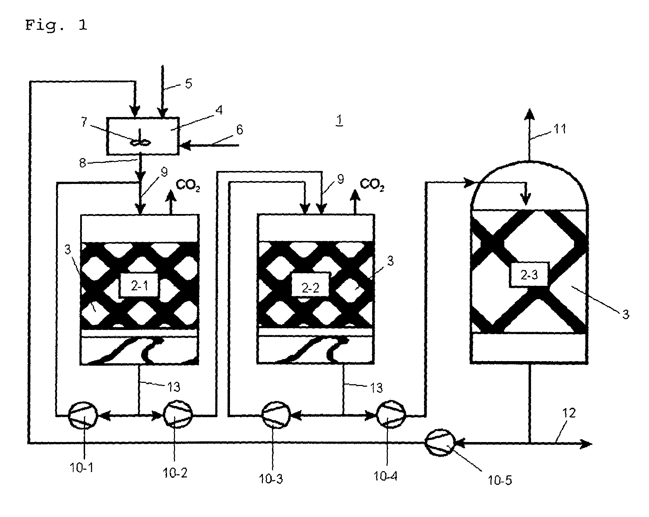

Im Folgenden wird die Erfindung anhand einer Zeichnung näher beschrieben. Im Einzelnen zeigt
Figur 1 eine schematische Darstellung der Biogasanlage.

Figur 1 zeigt eine schematische Darstellung der Biogasanlage 1. Diese Biogasanlage 1 kann zum Beispiel zur Biogasgewinnung aus Glyzerin beladenem Abwasser verwendet werden.

Die in Figur 1 dargestellt Biogasanlage 1 besteht aus drei Festbettreaktoren 2, wobei die Festbettreaktoren 2-1 und 2-2 als Hydrolysefestbettreaktoren und der Festbettreaktor 2-3 als Methanfestbettreaktor ausgebildet sind.

Die Festbettreaktoren 2 sind mit Füllkörpern 3 befüllt, auf denen je nach Verwendung des Festbettreaktors 2 Hydrolysebakterien oder Methanbakterien immobilisiert sind.

Des Weiteren besteht die Biogasanlage 1 aus einem Mischbehälter 4, der einen Einlass 5 für organisch beladenes Abwasser und eine Belüftung 6 aufweist. In dem Mischbehälter befindet sich ein Rührer 7 oder eine andere zum Mischen der eingebrachten Substanzen geeignetes Mischwerkzeug.

Weiterhin befindet sich an dem Mischbehälter 4 ein Auslass 8, durch den der Mischbehälter 4 entleert werden und das sich im Mischbehälter 4 befindliche Abwasser in einen ersten Hydrolysefestbettreaktor 2-1 überführt werden kann.

Das durch den Einlass 9 des Hydrolysefestbettreaktors 2-1 gelangte vorgemischte Abwasser passieren die Füllkörper 3 im Inneren des Reaktors und kann von dort mit Pumpen 10 in verschiede Kreislaufsysteme gepumpt werden. Durch die Pumpe 10-1 kann die Flüssigkeit wieder in den Einlass 9 des Hydrolysefestbettreaktors 2-1 gepumpt werden. Auf diese Weise ist es möglich, die Flüssigkeit mehrfach durch den Hydrolysefestbettreaktor 2-1 zu zirkulieren.

Es ist jedoch auch möglich, die Flüssigkeit mit der Pumpe 10-2 in den zweiten Hydrolysefestbettreaktor 2-2 zu pumpen.

In dem Hydrolysefestbettreaktor 2-2 wird die Flüssigkeit über Füllkörpern 3 geleitet, auf denen ebenfalls Hydrolysebakterien immobilisiert sind. Im zweiten Hydrolysefestbettreaktor 2-2 kann die Flüssigkeit durch ein Kreislaufsystem entweder mit der Pumpe 10-3 wieder in den Hydrolysereaktor 2-2 überführt und somit zirkuliert werden oder durch die Pumpe 10-4 in den Methanfestbettreaktor 2-3 gepumpt werden. Hierzu befindet sich an der Unterseite der Hydrolysefestbettreaktoren 2-1 und 2-2 ein Auslass 13.

Im unteren Bereich der Festbettreaktoren 2 befindet sich eine Gitterkonstruktion, durch die die Füllkörper 3 filterartig zurückgehalten werden, beziehungsweise auf der die Füllkörper 3 aufliegen, damit sie nicht ausgespült werden. Die Füllkörper 3 können jedoch auch durch andere Konstruktionen daran gehindert werden aus dem Festbettreaktor 2 herausgespült zu werden.

Die Hydrolysefestbettreaktoren 2-1 und 2-2 sind offen, so dass das bei der Hydrolysereaktion im Reaktor entstehende Kohlendioxid (CO₂) entweichen kann.

Der Methanreaktor 2-3 ist mit Füllkörpern 3 befüllt, auf den Methanbakterien immobilisiert sind. Das bei dieser Methanstufe entstehende Biogas kann dem Methanfestbettreaktor 2-3 durch einen Gasauslass 11 entnommen werden.

Die den Methanfestbettreaktor 2-3 durchlaufende Flüssigkeit kann durch einen weiteren Kreislauf mit der Pumpe 10-5 als Kreislaufwasser wieder in den Mischbehälter 4 überführt oder der Biogasanlage 1 als entfrachtetes Abwasser am Ablauf 12 entzogen werden.

Das Verfahren zur Herstellung von Biogas besteht aus zwei Hydrolysestufen, die nacheinander von dem Abwasser durchflossen werden. Jede der Hydrolysestufen ist als Festbettreaktor 2 ausgeführt und mit Füllkörpern 3 befüllt, wobei zum Beispiel Holzhackschnitzel als Füllkörper 3 verwendet werden können. Die Holzhackschnitzel wurden vor der Inbetriebnahme des Prozesses mit autochthon gewonnenen Hydrolysebakterien beimpft. Beide Hydrolysestufen sind offen, so dass Kohlendioxid entweichen kann.

Das organische, hier mit Glyzerin beladene Abwasser wird mit Kreislaufwasser aus dem Ablauf der Methanstufe gemischt. Gleichzeitig erfolgt eine Belüftung zur Abtötung bzw. Inhibierung der aus der Methanstufe mitgeschleppten Methanbakterien. Das vorgemischte Abwasser wird dann der ersten Hydrolysestufe zugegeben.

In den einzelnen Hydrolysestufen erfolgt mit Hilfe der Pumpen 10-1 und 10-3 eine Rückführung der Flüssigkeit, die somit mehrfach über die Füllkörper der jeweiligen Reaktoren geführt wird. Die Kopplung zwischen der ersten und der zweiten Hydrolysestufe erfolgt mittels einer Pumpe 10-2, die einen Flüssigkeitsstrom aus dem Rückführungskreislauf der ersten Hydrolysestufe in den Zulauf der zweiten Hydrolysestufe fördert.

Die Hydrolysestufen sollen so ausgelegt werden, dass die Verweilzeiten in jeder der Stufen etwa 24 Stunden beträgt.

Mit Hilfe der Pumpe 10-4 wird ein nunmehr mit Hydrolyseprodukten, vorwiegend organischen Säuren, beladender Flüssigkeitsstrom aus dem Rückführungskreislauf der zweiten Hydrolysestufe in die Methanstufe transportiert. Die Methanstufe besteht ebenfalls aus dem Festbettreaktor 2, der mit Kunststofffüllkörpern befüllt ist, auf denen Methanbakterien immobilisiert sind.

In dieser Methanstufe werden die Hydrolyseprodukte in Biogas umgewandelt, wobei aus einem Liter des als Input zugegebenen Glyzerin beladenen Abwassers bis zu 1000 Liter Biogas mit einem Methangehalt von > 60 % gewonnen werden kann. In der Regel ist dazu nur eine einzige Durchströmung des Methanfestbettreaktors bei einer Verweilzeit von maximal 20 Stunden erforderlich.

Das die Methanstufe verlassende, nunmehr von organischen Stoffen weitestgehend entfrachtete Abwasser wird zu einem Teil als Kreislaufwasser in den Prozess zurückgeführt, der verbleibende Teil wird entsorgt und kann üblicherweise ohne weitere Behandlung in die Kanalisation eingeleitet werden.

### Bezugszeichenliste:

- 1: Biogasanlage
- 2: Festbettreaktor
- 2-1: erster Hydrolysefestbettreaktor
- 2-2: zweiter Hydrolysefestbettreaktor
- 2-3: Methanfestbettreaktor
- 3: Füllkörper
- 4: Mischbehälter
- 5: Einlass
- 6: Belüftung
- 7: Rührer
- 8: Auslass des Mischbehälters
- 9: Einlass eines Hydrolysefestbettreaktors
- 10: Pumpe
- 10-1: Pumpe des ersten Rückführungskreislaufes
- 10-2: Pumpe zum zweiten Hydrolysefestbettreaktor
- 10-3: Pumpe des zweiten Rückführungskreislaufes
- 10-4: Pumpe zum Methanfestbettreaktor
- 10-5: Pumpe zur Rückführung des Kreislaufwassers
- 11: Gasauslass
- 12: Ablauf des Methanfeststoffreaktors
- 13: Auslass des Methanfestbettreaktors

## Patentansprüche

1. Verfahren zur Herstellung von Biogas aus organisch beladenen Flüssigkeiten, wobei
a) man die organisch beladenen Flüssigkeiten in einem Mischbehälter (4) belüftet und anschließend in einen Hydrolysefestbettreaktor (2-1) überführt,
b) man eine Hydrolyse in mindestens einem Hydrolysefestbettreaktor (2-1) durchführt, der Füllkörper (3) enthält, wobei man die Flüssigkeit mehrfach über die Füllkörper (3) führt indem man die Flüssigkeit abpumpt und über einen Rückführungskreislauf wieder dem Hydrolysefestbettreaktor (2-1) zuführt oder die Flüssigkeit in weitere Hydrolysefestbettreaktoren (2-2) pumpt,
c) man einen Teilstrom der organisch beladenen Flüssigkeit aus dem Rückführungskreislauf in einen Methanfestbettreaktor (2-3) pumpt, der Füllkörper (3) enthält und zu Biogas vergärt.

2. Verfahren nach Anspruch 1, **dadurch gekennzeichnet, dass** man die organisch beladene Flüssigkeit im Mischbehälter (4) mit Kreislaufwasser versetzt.

3. Verfahren nach Anspruch 2, **dadurch gekennzeichnet, dass** man das Kreislaufwasser aus dem Ablauf (12) des Methanfestbettreaktors (2-3) in den Mischbehälter (4) pumpt.

4. Verfahren nach einem der Ansprüche 1 bis 3, **dadurch gekennzeichnet, dass** man als Füllkörper (3) Holzhackschnitzel und/oder Kunststofffüllkörper und/oder Bambusringe verwendet.

5. Verfahren nach einem der Ansprüche 1 bis 4, **dadurch gekennzeichnet, dass** man die Füllkörper (3) in den Hydrolysefestbettreaktoren (2-1, 2-2) mit Hydrolysebakterien beimpft.

6. Verfahren nach einem der Ansprüche 1 bis 5, **dadurch gekennzeichnet, dass** man die Füllkörper (3) in dem Methanfestbettreaktor (2-3) mit Methanbakterien beimpft.

7. Verfahren nach einem der Ansprüche 1 bis 6, **dadurch gekennzeichnet, dass** man die Hydrolysefestbettreaktoren (2-1, 2-2) als offenes System betreibt.

8. Verfahren nach einem der Ansprüche 1 bis 7, **dadurch gekennzeichnet, dass** man die Hydrolyse im Hydrolysefestbettreaktor (2-3) unter schwach aeroben Bedingungen betreibt.

9. Biogasanlage (1) für ein Verfahren nach einem der vorhergehenden Ansprüche, bestehend aus einem Mischbehälter (4) mit Belüftung (6), mindestens einem Hydrolysefestbettreaktor (2-1, 2-2), Pumpen (10) und einem Methanfestbettreaktor (2-3).

10. Biogasanlage (1) nach Anspruch 9, **dadurch gekennzeichnet, dass** mehrere Hydrolysefestbettreaktoren (2-1, 2-2) in Reihe hintereinander betreibbar sind.

11. Biogasanlage (1) nach Anspruch 9 oder 10, **dadurch gekennzeichnet, dass** die Hydrolysefestbettreaktoren (2-1, 2-2) Füllkörper (3) enthalten, auf denen Hydrolysebakterien immobilisiert sind.

12. Biogasanlage (1) nach einem der Ansprüche 9 bis 11, **dadurch gekennzeichnet, dass** der Methanfestbettreaktor (2-3) Füllkörper (3) enthält, auf denen Methanbakterien immobilisiert sind.

13. Biogasanlage (1) nach einem der Ansprüche 9 bis 12, **dadurch gekennzeichnet, dass** die Füllkörper (3) Holzhackschnitzel und/oder Kunststofffüllkörper und/oder Bambusringe sind.

14. Biogasanlage (1) nach einem der Ansprüche 9 bis 13, **dadurch gekennzeichnet, dass** ein Leitungssystem mit Pumpen (10) den Mischbehälter (4), die Hydrolysereaktoren und den Methanreaktor miteinander verbinden.

15. Biogasanlage (1) nach einem der Ansprüche 9 bis 14, **dadurch gekennzeichnet, dass** die Hydrolysefestbettreaktoren (2-1, 2-2) einen Rückführungskreislauf mit einer Pumpe (10-1, 10-3) enthalten.

16. Biogasanlage (1) nach einem der Ansprüche 9 bis 15, **dadurch gekennzeichnet, dass** der Methanfestbettreaktor (2-3) einen Ablauf (12) besitzt.

17. Biogasanlage (1) nach einem der Ansprüche 1 bis 16, **dadurch gekennzeichnet, dass** der Methanfestbettreaktor (2-3) einen Kreislauf für Kreislaufwasser von dem Methanfestbettreaktor (2-3) zum Mischbehälter (4) mit einer Pumpe (10-5) besitzt.

18. Verwendung der Biogasanlage (1) nach einem der Ansprüche 9 bis 17 zur Herstellung von Biogas.

19. Verwendung des Verfahrens nach einem der Ansprüche 1 bis 8 zur Monovergärung von glyzerinhaltigem Abwasser.
